# EUROPEAN PATENT APPLICATION

(11) **EP 4 781 962 A1**
(43) Date of publication of application: **29.07.2026**
(21) Application number: 25154183.5
(22) Date of filing: 27.01.2025
(51) Int. Cl.: A61F 13/49

(54) **WASHABLE, REUSABLE UNDERWEAR**

(71) Applicant: snuggswear s.r.o., 60200 Brno (CZ)
(72) Inventor: Zahradnik, Tomas, 15400 Praha (CZ); Sejdova, Linda, 15200 Praha (CZ); Sobotka, Radek, 39701 Pisek (CZ); Simkova, Lenka, 39806 Mirovice (CZ)
(74) Representative: Zemanová, Veronika

(57) **Abstract**

Washable, reusable underwear comprising a gusset component (10) comprising an outer knitted layer (1) on which a barrier layer (2) is placed, on which an absorption layer (3) is placed, on which a transport layer (4) is placed. The absorption layer (3) comprises a knitted fabric comprising a viscose yarn in an amount of at least 60 wt. %, preferably at least 70 wt. %, wherein a plurality of at least two, preferably at least three distribution regions (12) are knitted in the absorption layer (3), which extend in the longitudinal direction of the gusset component (10) and comprise a polypropylene yarn.

## Description

### Technical Field

The invention relates to underwear, in particular to washable, reusable panties with an absorbent gusset or to washable, reusable, preferably close-fitting, men's underwear with an absorbent crotch gusset component. Said underwear is useful in particular in cases of incontinence and/or menstruation.

### Prior art

Disposable incontinence aids and disposable menstrual pads and tampons, usually made of non-woven fabrics, are known from the prior art. Their disadvantage is that they are uncomfortable to wear and that their use poses a great environmental burden.

Furthermore, washable, reusable panties intended for use during menstruation or discharge are known, as a replacement or supplement to disposable menstrual pads, tampons and cups.

The task of the present invention is to increase the comfort of wearing such washable, reusable panties, in particular to ensure rapid transport of moisture from the body surface, i.e. from the upper layer to the inner layers, and thus to provide a feeling of dryness on the skin surface. Another task is to retain the liquid in the inner layers even during prolonged, several-hour wear. In this case, the crotch gusset should have the smallest possible thickness so that it does not create noticeable contours under clothing, is comfortable and safe.

### Summary of Invention

The above aim is achieved by means of a washable, reusable underwear comprising a gusset component comprising an outer knitted layer on which a barrier layer is placed, on which an absorption layer is placed, on which a transport layer is placed, wherein the absorption layer comprises a knitted fabric comprising a viscose yarn in an amount of at least 60 wt. %, preferably at least 70 wt. %, wherein a plurality of at least two, preferably at least three distribution regions are knitted in the absorption layer, which extend in the longitudinal direction of the gusset component and comprise a polypropylene yarn.

Preferably, each of the distribution regions is formed by three to ten rows, preferably four to six rows, of knitted fabric comprising at least 80 wt. % polypropylene yarn, and/or the distribution regions are spaced twelve to thirty, preferably fifteen to twenty-five, rows of knitted fabric containing viscose yarn, and/or each of the distribution regions extends from the front edge to the rear edge of the absorption layer.

In a preferred embodiment of the absorption layer
- said viscose yarn has a linear weight of 10 to 40 tex, preferably 20 to 35 tex, and/or said viscose yarn is a yarn of staple fibers, preferably hollow or multi-channel fibers, and/or
- said polypropylene yarn in the distribution regions has a linear weight of 15 to 250 dtex, preferably 20 to 150 dtex.

It is also advantageous, when said knitted fabric contained in the absorption layer forms a plush or interlock weave and has a basis weight of 200 to 500, preferably 250 to 450, most preferably 280 to 420 g/m².

Preferably, said knitted fabric contained in the absorption layer is adjacent to the barrier layer, has a plush weave and basis weight of 200 to 500, preferably 250 to 450, most preferably 280 to 420 g/m², wherein the absorption layer further comprises another knitted fabric which is placed on said plush weave knitted fabric and which comprises a viscose yarn in an amount of at least 60 wt. %, more preferably at least 70 wt. %. For such case, said another knitted fabric in the absorption layer preferably forms an interlock or plush weave and comprises distribution regions which extend in the longitudinal direction of the gusset component and each of which is formed by one to ten rows comprising a polypropylene yarn.

According to a particularly advantageous embodiment the transport layer is formed by a knitted fabric containing polypropylene yarn forming a filet warp weave, wherein the polypropylene yarn content is greater than 80 wt. %, wherein the basis weight of the transport layer is preferably 50 to 140 g/m², preferably 70 to 110 g/m², and/or the polypropylene yarn has a linear weight of 70 to 150, preferably 90 to 134 dtex.

A preferred embodiment of the underwear comprises an additional hem of hydrophobic knitted fabric, which is sewn to the side edges of the transport layer and via the transport layer also to the absorption layer along their side edges, and which is folded around this stitching out from the area of the transport layer and the absorption layer, where it is sewn together with the barrier layer and the outer knitted layer.

Preferably, the underwear comprises a cover hem made of hydrophobic knitted fabric, which is sewn to the additional hem, to the outer knitted layer and via the outer knitted layer to the barrier layer, the cover hem surrounding the outer side edges of the additional hem, the barrier layer and the outer knitted layer, wherein preferably the additional hems and the cover hems are sewn using a hydrophobic yarn.

Preferably, the underwear has a front part and a back part, the side regions of which are connected to each other, preferably by stitching or in a seamless manner, and the lower regions of which are mutually interconnected by the gusset component.

### Brief Description of the Drawings

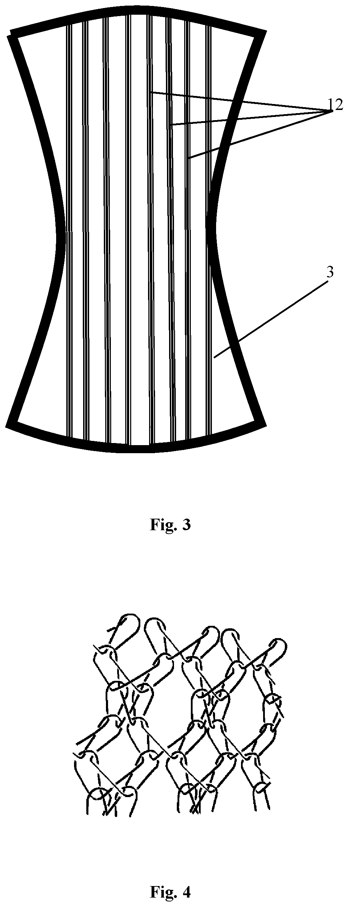

The invention is further described in more detail on the basis of exemplary embodiments and drawings, wherein Fig. 1 schematically shows a front view of panties, Fig. 2 shows a cross-section of a gusset component of an exemplary embodiment of panties, Fig. 3 shows a plan view of the gusset component of another exemplary embodiment of panties, Fig. 4 shows a filet weave, Fig. 5 a plush weave and Fig. 6 an interlock weave.

### Description of Exemplary Embodiments

Panties comprise a front part 20, a back part 30, the side regions of which are directly connected to each other by stitching or in a seamless manner, and the lower regions of which are connected to each other by a gusset component 10. The front part 20 and the back part 30 define - with their upper edges - an opening for torso, wherein together with the side edges of the gusset component they define openings for leg.

The gusset component 10 is a part which - when the panties are worn - is arranged adjacent the crotch. In this text, the word front is used to indicate the part or region that is closest to the lower abdomen, when the panties are worn, and the word back is used to indicate the part or region that is closest to the buttocks, when the panties are worn.

In a plan view, the gusset component 10 is narrowest in the central region and widens towards the front region and the back region.

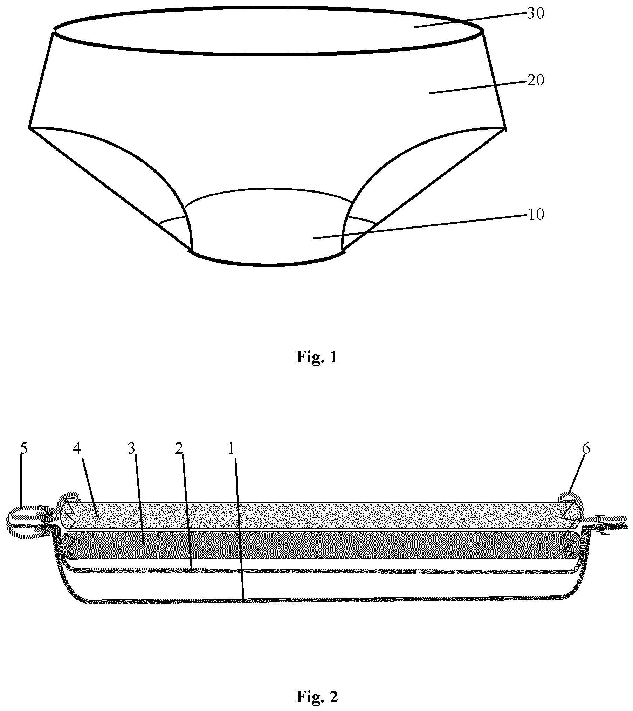

The gusset component 10 of the exemplary embodiment of the panties, which is shown in cross-section in Fig. 2, contains a transport layer 4, which is placed on an absorption layer 3, which is placed on a barrier layer 2, which is placed on an outer knitted layer 1.

The outer knitted layer 1 is made of recycled polyamide filaments (designation 7087) combined with in-woven elastane (a fiber with a high modulus of elasticity) in a plain covered weave. The outer knitted layer also forms the back part 30 and the front part 20 of the panties.

Alternatively, any other knitted fabrics suitable for the production of underwear and made of synthetic polymers or natural polymers, or a combination thereof, can be used for the outer knitted layer. For example, a suitable material for the outer knitted layer is a plain knitted fabric made of natural polymers, such as modal or tencel, with an addition of elastane, and a plain cotton knitted fabric made of long stapled cotton mixed with elastane.

The barrier layer 2 is made of a polyester or polyamide knitted fabric, which is provided with a polyurethane nanomembrane, with a total basis weight of approximately 50 g/m², of which 13 g/m² is for the nanomembrane.

It is generally suitable to use for the barrier layer a warp knitted fabric having a basis weight of preferably 15 to 85 g/m², preferably 20 - 40 g/m², while the nanomembrane having a basis weight of 12 to 15 g/m² is fixed to this supporting, preferably polyester, preferably warp, non-running knitted fabric. Such a barrier layer 2 is highly resistant to maintenance. It withstands up to 80 maintenance cycles of washing and drying process.

### Absorption layer - plush

According to an exemplary embodiment, the absorption layer 3 is a knitted fabric having a plush weave (so-called terry), in which the base (the smooth side) is formed by a polyester yarn of continuous filaments, which has 100/36 dtex, i.e. the yarn has a linear weight of 100 dtex and contains 36 filaments. The plush loop is formed by a yarn composed of staple viscose profiled fibers, having of a round, six-channel cross-section of the modal type and has a linear weight of 30 tex.

The absorption layer 3 contains distribution regions 12 in said knitted fabric, the distribution regions 12 being formed by rows of polypropylene yarn 110/33 dtex, i.e. a yarn with a linear weight of 110 dtex and containing 33 fibers. In this exemplary embodiment, in the distribution region 12, the warp and the loop are both knitted from this polypropylene yarn such that they always form 4 rows next to each other, which alternate with 20 rows of polyester and viscose, as described above. The distribution regions 12 ensure distribution of liquid in the longitudinal direction of the gusset, i.e. in the direction from the central area to its front area and to its rear area. Thanks to this, liquid coming to the central area hardly spills to the sides, where it might flow around the edge before it is fully absorbed by the absorption layer 3.

Viscose yarn constitutes 78 wt. % of the knitted fabric, polyester yarn constitutes 14 wt. % and polypropylene yarn constitutes 8 wt. %.

The knitted fabric forming the absorption layer 3 has a basis weight of 400 g/m2, with a density of 14 rows/cm and 9 columns/cm.

In case lower absorption properties of the absorption layer 3 are required, it is possible to create the knitted fabric described above, but with the difference that it has a basis weight of 300 g/m² and for the plush loop, viscose yarn from staple fibers of the modal type is used, which has a linear weight of 20 tex. In such a case, viscose yarn constitutes 74 wt. % of the knitted fabric, polyester yarn constitutes 18 wt. % and polypropylene yarn constitutes 8 wt. %.

In general, a plush weave knitted fabric for the absorption layer 3 can be a knitted fabric with a basis weight of 200 to 500, preferably 250 to 450, preferably 300 to 400 g/m², in which a yarn having a linear weight of 10 to 40 tex, preferably 18 to 35 tex is used for the plush loop, the yarn material being made of a natural cellulose-based polymer, preferably viscose, which can be of various types, for example modal, bamboo, micromodal, etc., and the smooth base of the knitted fabric is made of any suitable yarn, preferably polyester yarn, preferably with a linear weight of 50 to 150 dtex. Such knitted fabric is structurally adapted for directional moisture conduction, namely by containing distribution regions 12, i.e. plurality of rows of polypropylene yarn. Preferably, each distribution region 12 comprises one to seven rows arranged side by side, preferably three to five rows of polypropylene yarn knitted in a smooth plush weave, with preferably 8 to 50, preferably 15 to 30 rows of viscose and polyester between these distribution regions 12. Considering a gusset design, such knitted structure is suitable for maximizing the absorbency of the knitted fabric while limiting absorption at the narrowest area of the crotch gusset.

### Absorption layer - interlock

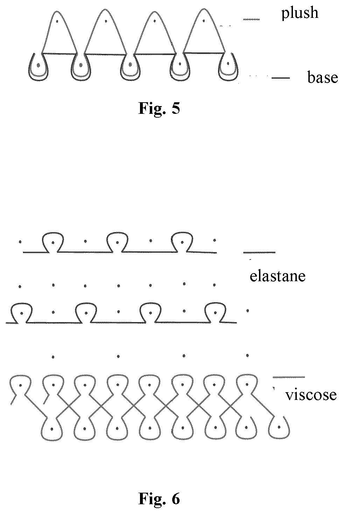

According to another exemplary embodiment, the absorption layer 3 comprises a knitted fabric having a basis weight of 300 g/m² forming an interlock weave, in which a viscose yarn comprising a staple profiled viscose fibre and having a linear weight of 30 tex is combined with a polypropylene filament yarn, said polypropylene yarn having a linear weight of 33/6 dtex, and with elastane (Fig. 6). The density of the knit is 15 rows/cm and 12 columns/cm. In this knitted fabric too, there are always several distribution regions 12, which are formed by rows of polypropylene yarn.

The viscose yarn constitutes 93 wt. % of the knitted fabric, the polypropylene yarn constitutes 5 wt. % and the elastane constitutes 2 wt. %.

Another embodiment of this knitted fabric differs from the knitted fabric described above in that it has a basis weight of 340 g/m² and the polypropylene yarn has 110/33 dtex. The viscose yarn constitutes 96 wt. % of the knitted fabric, the polypropylene yarn constitutes 2 wt. % and the elastane constitutes 2 wt. %.

According to another alternative embodiment, the viscose yarn, or the yarn within the interlock weave, can be combined with a polyamide yarn.

In general, a knitted fabric with an basis weight of 200 to 500, preferably 250 to 450, more preferably 300 to 400 g/m² can be used as an interlock weave knitted fabric for the absorption layer 3, in which a viscose yarn with a linear weight of 10 to 40 tex, preferably 20 to 35 tex is used, and the viscose can be of various types, for example modal, bamboo, etc. and wherein said viscose yarn is combined in an interlock weave with a polyester or polyamide yarn, preferably having a linear weight of 10 to 100 dtex. Said knitted fabric is structurally adapted for directional moisture conduction, namely by containing several spaced-apart absorbent areas 12 in the form of rows of polypropylene yarn.

### Combination of absorption layers

The absorption layer 3 can be formed from several sub-layers to increase its absorption capacity by combining these sub-layers.

For example, it is advantageous to use for the absorption layer 3 one of the above-described knitted fabrics forming a plush weave combined with one of the above-described knitted fabrics forming an interlock weave, which thus increases the volume of absorbed liquid and at the same time ensures very good permeability of the liquid into said plush layer.

Alternatively, it is possible to combine, for example, a pair of knitted fabrics forming a plush weave or a pair of knitted fabrics forming an interlock weave.

Knitted fabrics with plush and interlock weaves have been described above, but in less advantageous embodiments it is also possible to use other weaves of knitted fabrics, for example a rib-structure weave or fleece weave, with a distribution region 12 in the form of several rows of polypropylene yarn.

The transport layer 4 is made of polypropylene knitted fabric forming a filet weave, with a basis weight of 95 g/m². Said polypropylene knitted fabric is made of polypropylene yarn from continuous filaments, specifically from yarn 56/33 x 2 dtex, i.e. the yarns contain two yarns, each of which contains 33 filaments and each has a linear weight of 56 dtex.

This knitted fabric preferably has holes in the filet weave, the largest dimension of which is 1.5 mm to 2 mm, when the knitted fabric is in a relaxed/unstretched state.

The gusset component 10 is additionally provided on the inside of the panties with an additional hem 6 and a cover hem 5.

Both hems 5, 6 are made of a flexible knitted fabric provided with a hydrophobic treatment preventing moisture leakage and ensuring close fit of at least the edges of the panties. Hems 5, 6 contain a supporting polyamide knit on which a water-impermeable hydrophobic treatment is fixed, in this exemplary embodiment the basis weight of this polyamide knitted fabric is 155 g/m² with an elastic fiber content of 15 wt. %. It is generally suitable that the elastic fiber content in the knitted fabric of the hems 5, 6 is at least 10 wt. %. The hydrophobic treatment can provided by preparations guaranteeing a hydrophobic effect with high resistance to washing, for example a preparation based on hyperbranched polymers in a hydrocarbon matrix, for example the preparation RUCO-DRY ECO DHF from Rudolf GmbH.

As may be inferred from Fig. 2, the connection of the individual components of the gusset component is as follows: The transport layer 4 and the absorption layer 3 have essentially the same shape and size in plan view and are placed on top of each other and along each of their side edges and along the front and back edges they are sewn together with an additional hem 6, which extends beyond those layers on the sides of the gusset there. This protruding area of the additional hem 6 is sewn outside the area of the transport layer 4 and the absorption layer 3 to the edges of the barrier layer 2 and the outer knitted layer 1 (right side of Fig. 2), wherein this sewing is carried out over the cover hem 5, which forms edgings of the entire openings for legs, and this sewing is carried out with a hydrophobic sewing yarn. The additional hem 6 on the front and back edges of the gusset component 10 is bent towards the outer knitted layer 1 and sewn to it with a suitable sewing stitch.

The gusset component 10 can be formed with the following layering for different levels of load, whereby in the following text the number of stars indicates the level of load from the smallest number of stars for the smallest expected load to the highest number of stars for the highest expected load of menstrual or incontinence panties:
* (absorption level 1) - transport layer 4 formed of a knitted fabric forming a filet weave of polypropylene yarn - absorption layer 3 with a basis weight of 300 g/m2 formed of an interlock knitted fabric of viscose yarn and with inserted rows of polypropylene yarn - barrier layer 2 of a knitted fabric with a water-impermeable nanomembrane - outer knitted layer 1
** (absorption level 2) - transport layer 4 made of knitted fabric with a filet weave of polypropylene yarn - absorption layer 3 made of knitted fabric with a basis weight of 340 g/m² forming a plush weave of viscose yarn and polyester yarn and with inserted rows of polypropylene yarn - barrier layer 2 made of knitted fabric with a water-impermeable nanomembrane - outer knitted layer 1
*** (absorption level 3 - absorption measured by the internal method 60 ml) - transport layer 4 made of knitted fabric forming a filet weave of polypropylene yarn - absorption layer 3 made of knitted fabric forming a plush weave of viscose yarn and polyester yarn and with inserted rows of polypropylene yarn, the knitted fabric having a basis weight of 400 g/m2 - barrier layer 2 made of knitted fabric with a water-impermeable nanomembrane - outer knitted layer 1
**** (absorption level 4) - transport layer 4 made of knitted fabric forming filet weave made of polypropylene yarn - absorption layer 3 made of two sub-layers, namely knitted fabric forming interlock weave made of viscose yarn and inserted rows of polypropylene yarn, the knitted fabric having a basis weight of 300 g/m², and a plush layer having a basis weight of 300 g/m² - (total basis weight 600 g/m²) - barrier layer 2 made of knitted fabric with a water-impermeable nanomembrane - outer knitted layer 1
***** (absorption level 5) - transport layer 4 made of knitted fabric forming filet weave made of polypropylene yarn - absorption layer 3 made of two sub-layers, namely knitted fabric with a basis weight of 300 g/m² forming interlock weave made of viscose yarn and inserted rows of polypropylene yarn and knitted fabric with a basis weight of 400 g/m² with a plush weave of viscose yarn and polyester yarn with inserted rows of polypropylene yarn (total basis weight of the absorption layer 3 is 700 g/m²) - barrier layer 2 made of knitted fabric with a water-impermeable membrane - outer knitted layer 1
****** (absorption level 6) - transport layer 4 made of knitted fabric forming a filet weave made of polypropylene yarn - absorption layer 3 made of two partial layers of knitted fabric with a basis weight of 400 g/m² forming a plush weave made of viscose yarn and polyester yarn with inserted rows of polypropylene yarn (total basis weight of absorption layer 3 is 800 g/m²) - barrier layer 2 made of knitted fabric with a water-impermeable membrane - outer knitted layer 1

The linear weight is provided based on measurements according to the ČSN EN ISO 1973 standard.

The basis weight is provided based on measurements according to the ČSN EN 12127 standard.

The number of rows and columns of the knitted fabric per cm is indicated based on measurements according to the ČSN EN 14 971 standard. The data on the number of rows and columns are stated as indicative values.

The absorption layer 3 is preferably treated with an antibacterial / biocidal preparation, for example based on argentium - sodium - hydrogen zirconium phosphate.

The terms knitted fabric and knit are used in this application with the same meaning.

The term polypropylene yarn in this text means a yarn containing at least 80 wt. % polypropylene. The term viscose yarn in this text means a yarn containing at least 80 wt. % viscose. The term polyester yarn in this text means a yarn containing at least 80 wt. % polyester.

Although a number of exemplary embodiments have been described, it is apparent that other possible alternatives to these embodiments will readily occur to one skilled in the art. Therefore, the scope of the invention is not limited to these exemplary embodiments, but rather is defined by the appended claims.

## Claims

1. Washable, reusable underwear comprising a gusset component (10) comprising an outer knitted layer (1) on which a barrier layer (2) is placed, on which an absorption layer (3) is placed, on which a transport layer (4) is placed, **characterised in that** the absorption layer (3) comprises a knitted fabric comprising a viscose yarn in an amount of at least 60 wt. %, preferably at least 70 wt. %, wherein a plurality of at least two, preferably at least three distribution regions (12) are knitted in the absorption layer (3), which extend in the longitudinal direction of the gusset component (10) and comprise a polypropylene yarn.

2. Underwear according to claim 1, **characterised in that** each of the distribution regions (12) is formed by three to ten rows, preferably four to six rows, of knitted fabric comprising at least 80 wt. % polypropylene yarn, and/or the distribution regions (12) are spaced twelve to thirty, preferably fifteen to twenty-five, rows of knitted fabric containing viscose yarn, and/or each of the distribution regions extends from the front edge to the rear edge of the absorption layer (3).

3. Underwear according to claim 1 or 2, **characterized in that** in the absorption layer (3)
- said viscose yarn has a linear weight of 10 to 40 tex, preferably 20 to 35 tex, and/or said viscose yarn is a yarn of staple fibers, preferably hollow or multi-channel fibers, and/or
- said polypropylene yarn in the distribution regions (12) has a linear weight of 15 to 250 dtex, preferably 20 to 150 dtex.

4. Underwear according to any of claims 1 to 3, **characterized in that** said knitted fabric contained in the absorption layer (3) forms a plush or interlock weave and has a basis weight of 200 to 500, preferably 250 to 450, most preferably 280 to 420 g/m².

5. Underwear according to any of claims 1 to 3, **characterized in that** said knitted fabric contained in the absorption layer (3) is adjacent to the barrier layer (2), has a plush weave and basis weight of 200 to 500, preferably 250 to 450, most preferably 280 to 420 g/m², wherein the absorption layer (3) further comprises another knitted fabric which is placed on said plush weave knitted fabric and which comprises a viscose yarn in an amount of at least 60 wt. %, more preferably at least 70 wt. %.

6. Underwear according to claim 5, **characterized in that** said another knitted fabric in the absorption layer (3) forms an interlock or plush weave and comprises distribution regions (12) which extend in the longitudinal direction of the gusset component (10) and each of which is formed by one to ten rows comprising a polypropylene yarn.

7. Underwear according to any one of claims 1 to 6, **characterized in that** the transport layer (4) is formed by a knitted fabric containing polypropylene yarn forming a filet warp weave, wherein the polypropylene yarn content is greater than 80 wt. %, wherein the basis weight of the transport layer (4) is preferably 50 to 140 g/m², preferably 70 to 110 g/m², and/or the polypropylene yarn has a linear weight of 70 to 150, preferably 90 to 134 dtex.

8. Underwear according to any one of claims 1 to 7, **characterized by** comprising an additional hem (6) of hydrophobic knitted fabric, which is sewn to the side edges of the transport layer (4) and via the transport layer (4) also to the absorption layer (3) along their side edges, and which is folded around this stitching out from the area of the transport layer (4) and the absorption layer (3), where it is sewn together with the barrier layer (2) and the outer knitted layer (1).

9. Underwear according to claim 8, **characterized by** comprising a cover hem (5) made of hydrophobic knitted fabric, which is sewn to the additional hem (6), to the outer knitted layer (1) and via the outer knitted layer (1) to the barrier layer (2), the cover hem (5) surrounding the outer side edges of the additional hem (6), the barrier layer (2) and the outer knitted layer (1), wherein preferably the additional hems (6) and the cover hems (5) are sewn using a hydrophobic yarn.

10. Underwear according to any one of claims 1 to 9, **characterized in that** it has a front part (20) and a back part (30), the side regions of which are connected to each other, preferably by stitching or in a seamless manner, and the lower regions of which are mutually interconnected by the gusset component (10).

11. Underwear according to any one of claims 1 to 10, **characterized in that** the barrier layer (2) is formed by a warp knitted fabric, preferably a polyester or polyamide warp knitted fabric, having a basis weight of 15 to 85 g/m², preferably 20 - 40 g/m², on which a nanomembrane, preferably a polyurethane nanomembrane, with a basis weight of 12 to 15 g/m², is fixed.

12. Underwear according to any one of claims 1 to 11, **characterized in that** said knitted fabric and/or said further knitted fabric in the absorption layer (3) has a plain plush weave and comprises a polyester yarn which forms the smooth side of the plush weave.
